(19) Europäisches Patentamt
European Patent Office
Office européen des brevets

(11) **EP 3 895 608 A1**

(12) **EUROPEAN PATENT APPLICATION**
published in accordance with Art. 153(4) EPC

(43) Date of publication:
**20.10.2021 Bulletin 2021/42**

(51) Int Cl.:
**A61B 5/022** (2006.01)

(21) Application number: **18942666.1**

(86) International application number:
**PCT/JP2018/045306**

(22) Date of filing: **10.12.2018**

(87) International publication number:
**WO 2020/121377 (18.06.2020 Gazette 2020/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **National University Corporation Tokai National Higher Education and Research System Nagoya-shi, Aichi 464-8601 (JP)**

• **Laview Corporation Nagoya-shi, Aichi 464-0814 (JP)**

(72) Inventors:
• **MATSUMOTO Takeo Nagoya-shi Aichi 464-8601 (JP)**
• **MASUDA Hiroshi Nagoya-shi Aichi 464-0814 (JP)**

(74) Representative: **Seemann & Partner Patentanwälte mbB Raboisen 6 20095 Hamburg (DE)**

(54) **BIOLOGICAL INFORMATION MEASUREMENT DEVICE**

(57)     In a biological information measurement device 1, an internal pressure controller 30 increases or decreases an internal pressure of a compression band 10 wrapped around a part of a living body. A time-series data acquisition unit 32 acquires time-series data of the volume and the internal pressure of the compression band 10 while the internal pressure of the compression band 10 increases or decreases. A relationship deriving unit 34 derives a relationship between the volume and the internal pressure of the compression band 10 based on the time-series data. A determination unit 36 differentiates the relationship between the volume and the internal pressure with respect to the internal pressure to derive a first differential value, differentiates the first differential value with respect to time or the internal pressure to derive a second differential value, and determines the internal pressure at the peak of the second differential value as the blood pressure of the living body.

FIG.1

**Description**

TECHNICAL FIELD

The present disclosure relates to a biological information measurement device that measures biological information based on information obtained by a compression band wrapped around a part of a living body.

BACKGROUND ART

In recent years, there is an increasing demand for measuring biological information such as blood pressure and an index indicating an endothelial function of a blood vessel. Patent Literature 1 discloses an electronic sphygmomanometer that measures blood pressure by an oscillometric method.

Meanwhile, as an index indicating an endothelial function of a blood vessel, a dilation rate of a vascular diameter obtained by flow-mediated dilation (FMD) measurement is known. In the FMD measurement, a vascular diameter of an arm of a subject in a resting state is measured, a vascular diameter after the arm is subjected to avascularization and released is measured, and a dilation rate of the vascular diameter is calculated from these values. The vasodilation response occurs primarily in arteries rich in smooth muscle. When the endothelial function of the blood vessel decreases due to arteriosclerosis or the like, the dilation rate of the vascular diameter decreases.

Patent Literature

[Patent Literature 1] JP2015-9044 A

SUMMARY OF INVENTION

TECHNICAL PROBLEM

The oscillometric method is a method of measuring blood pressure using pulsation of a blood vessel accompanying pulsation of a heart, and determining a systolic blood pressure and a diastolic blood pressure based on a change in amplitude of a pulse wave. Since the algorithm for determining the blood pressure is determined by an empirical method, the actual blood pressure and the measured value may be different from each other, and there is room for improvement in the accuracy of the measured value.

Further, in general FMD measurement, since the diameter of a blood vessel is measured using an ultrasound image of the blood vessel with one large artery as a measurement target, it is necessary for an operator to be skilled in acquiring the ultrasound image and acquiring the inner diameter of the blood vessel from the image, and it is difficult to shorten the measurement time. Furthermore, although the vascular volume increases due to the vasodilation response, a change in the diameter of the blood vessel is measured. From these, even in the same subject, when the vascular diameter or measurement position of the blood vessel to be measured changes, the obtained dilation rate of the vascular diameter may change.

In this way, it is desired to improve the measurement accuracy of the biological information by simple measurement.

The present disclosure has been made in view of such a situation, and an object of the present disclosure is to provide a biological information measurement device capable of accurately and easily measuring biological information.

SOLUTION TO PROBLEM

In order to solve the above problem, a biological information measurement device according to an aspect of the present disclosure includes: an internal pressure controller that increases or decreases an internal pressure of a compression band wrapped around a part of a living body; a time-series data acquisition unit that acquires time-series data of a volume and an internal pressure of the compression band while the internal pressure of the compression band increases or decreases; a relationship deriving unit that derives a relationship between the volume and the internal pressure of the compression band based on the time-series data; and a determination unit that differentiates the relationship with respect to the internal pressure to derive a first differential value, differentiates the first differential value with respect to time or the internal pressure to derive a second differential value, and determines an internal pressure at a peak of the second differential value as a blood pressure of the living body.

Another aspect of the present disclosure is also a biological information measurement device. This device includes: an internal pressure controller that increases an internal pressure of a compression band wrapped around a part of a living body, maintains the internal pressure at an avascularization pressure at which the part of the living body is subjected to avascularization, and then lowers the internal pressure to a hold pressure at which avascularization is released; a time-series data acquisition unit that acquires time-series data of a volume and the internal pressure of the compression band while the internal pressure of the compression band increases or decreases; a relationship deriving unit that derives a relationship between the volume and the internal pressure of the compression band based on the time-series data; a first fitting unit that fits a first function to the relationship in a range in which the internal pressure is higher than a diastolic blood pressure of the living body; a second fitting unit that fits a second function to the relationship in a range in which the internal pressure is equal to or higher than a predetermined pressure higher than the hold pressure and equal to or lower than the diastolic blood pressure; a vascular volume acquisition unit that acquires, as a vascular volume, a difference between a value of the first function at the hold pressure and a hold volume that is a value of the second function at the hold pressure; a change amount acquisition unit that acquires an amount of change in the internal pressure of the compression band after the internal pressure of the compression band reaches the hold pressure; and a dilation rate deriving unit that derives a dilation rate of a vascular diameter or the vascular volume of the living body based on the hold pressure, the hold volume, the vascular volume, and the amount of change in the internal pressure.

Still another aspect of the present disclosure is also a biological information measurement device. This device includes: an internal pressure controller that increases an internal pressure of a compression band wrapped around a part of a living body, maintains the internal pressure at an avascularization pressure at which the part of the living body is subjected to avascularization, and then lowers the internal pressure to a hold pressure at which avascularization is released; a time-series data acquisition unit that acquires time-series data of a volume and an internal pressure of the compression band while the internal pressure of the compression band increases or decreases; a relationship deriving unit that derives a relationship between the volume and the internal pressure of the compression band based on the time-series data; a fitting unit that fits a function to the relationship in a range in which the internal pressure is higher than a diastolic blood pressure of the living body; a vascular volume acquisition unit that acquires, as a vascular volume, a difference between a value of the function at the hold pressure and a hold volume that is a volume at the hold pressure in the relationship; a change amount acquisition unit that acquires an amount of change in the internal pressure of the compression band after the internal pressure of the compression band reaches the hold pressure; and a dilation rate deriving unit that derives a dilation rate of a vascular diameter or the vascular volume of the living body based on the hold pressure, the hold volume, the vascular volume, and an amount of change in the internal pressure.

Note that arbitrary combinations of the above components and modifications of the expressions of the present disclosure among methods, apparatuses, systems, recording media, computer programs, and the like are also effective as aspects of the present disclosure.

ADVANTAGEOUS EFFECTS OF INVENTION

According to the present disclosure, biological information can be accurately and easily measured.

BRIEF DESCRIPTION OF DRAWINGS

Fig. 1 is a diagram illustrating a configuration of a biological information measurement device according to a first embodiment.
Fig. 2 is a diagram illustrating a relationship between a volume and an internal pressure of a compression band in Fig. 1.
Fig. 3 is a diagram illustrating a configuration of a biological information measurement device according to a second embodiment.
Fig. 4 is a diagram illustrating a temporal change in an internal pressure of a compression band in Fig. 3.
Fig. 5 is a diagram illustrating a relationship between a volume and the internal pressure of the compression band in Fig. 3, and a first function and a second function.
Fig. 6 is a diagram illustrating a temporal change of the internal pressure of the compression band from a hold pressure, acquired by a change amount acquisition unit in Fig. 3 and a characteristic of the compression band.
Fig. 7 is a diagram illustrating a configuration of a biological information measurement device according to a third embodiment.

DESCRIPTION OF EMBODIMENTS

First Embodiment

Before a first embodiment is specifically described, an outline will be first described. The first embodiment relates to a biological information measurement device that measures blood pressure as biological information. As described above, in the sphygmomanometer by the oscillometric method, there is room for improvement in the accuracy of the measured value. Further, since the shape of the pulse wave affects the measurement accuracy, there is an appropriate increase rate or decrease rate of the internal pressure of the compression band for acquiring the pulse wave, and it is difficult to shorten the measurement time without deteriorating the measurement accuracy. Furthermore, in a case of a patient with a large amount of bleeding, a patient in a heart failure state, and the like, the blood pressure decreases and the pulsation of the blood vessel becomes weak. It is difficult to measure the blood pressure of such a patient by the oscillometric method.

In recent years, the number of cases of applying an artificial heart to heart failure patients is increasing. The artificial heart is mainly of an axial flow pump type which is inexpensive and easy to handle. In a case of a subject who uses this type of artificial heart, there is no pulsation of blood vessels, so that the blood pressure cannot be measured by the oscillometric method. It is also desired to non-invasively and simply measure the blood pressure of the subject with weak or no pulsation of the blood vessel as described above.

Therefore, in the present embodiment, a set of the volume and the internal pressure of the compression band is sequentially acquired while the internal pressure of the compression band wrapped around the upper arm or the like of the subject decreases. Then, a relationship between the volume and the internal pressure is derived from the acquired data, and the blood pressure is determined based on a peak position of a differential result of the relationship between the volume and the internal pressure.

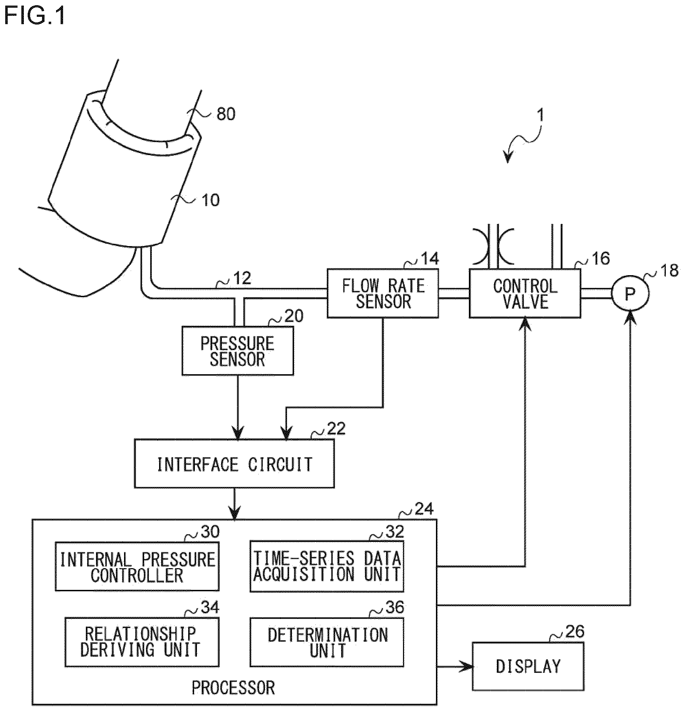

Fig. 1 illustrates a configuration of a biological information measurement device 1 according to the first embodiment. The biological information measurement device 1 can also be referred to as a blood pressure measurement device. The biological information measurement device 1 includes a compression band 10, a tube 12, a flow rate sensor 14, a control valve 16, an air pump 18, a pressure sensor 20, an interface circuit 22, a processor 24, and a display 26.

The compression band 10 has an expansion bag (not illustrated) and is wrapped around a part of a living body as a subject, for example, an upper arm 80. The compression band 10 is connected to the air pump 18 via the tube 12. The flow rate sensor 14 and the control valve 16 are provided in the middle of the tube 12. The flow rate sensor 14 is provided closer to the compression band 10 than the control valve 16.

The control valve 16 adjusts the pressure of the air pumped from the air pump 18 and supplies the air to the compression band 10 or discharges the air from the compression band 10, thereby adjusting the internal pressure of the compression band 10.

The flow rate sensor 14 is, for example, a hot wire anemometer, and detects the flow rate of the air supplied to the compression band 10 or the flow rate of the air discharged from the compression band 10.

The pressure sensor 20 is also connected to the compression band 10 via the tube 12. The pressure sensor 20 detects the internal pressure of the compression band 10.

The flow rate of the air detected by the flow rate sensor 14 and the internal pressure of the compression band 10 detected by the pressure sensor 20 are supplied to the processor 24 via the interface circuit 22 including an A/D converter.

The processor 24 includes an internal pressure controller 30, a time-series data acquisition unit 32, a relationship deriving unit 34, and a determination unit 36. The configuration of the processor 24 can be realized by a CPU, a memory, or another LSI of an arbitrary computer in terms of hardware, and is realized by a program loaded in a memory or the like in terms of software, but here, functional blocks realized by cooperation thereof are illustrated. Therefore, it is understood by those skilled in the art that these functional blocks can be realized in various forms by only hardware, only software, or a combination thereof.

The internal pressure controller 30 controls the control valve 16 and the air pump 18 based on the internal pressure of the compression band 10 detected by the pressure sensor 20 to increase or decrease the internal pressure of the compression band 10. The internal pressure controller 30 supplies air to the compression band 10 to increase the internal pressure, and discharges the air from the compression band 10 to decrease the internal pressure.

When starting blood pressure measurement, the internal pressure controller 30 increases the internal pressure of the compression band 10 to a target maximum pressure higher than the systolic blood pressure of the subject and then decreases the internal pressure to a target minimum pressure lower than the diastolic blood pressure of the subject. The target maximum pressure and the target minimum pressure can be appropriately determined by experiments or the like. The target maximum pressure may be, for example, about 180 mmHg, and the target minimum pressure may be, for example, about 20 mmHg.

The time-series data acquisition unit 32 acquires time-series data of a set of the volume and the internal pressure of the compression band 10 while the internal pressure of the compression band 10 decreases from the target maximum pressure, based on the flow rate of the air detected by the flow rate sensor 14 and the internal pressure detected by the pressure sensor 20. The time-series data also includes acquisition times of the volume and the internal pressure. The time-series data acquisition unit 32 outputs the time-series data to the relationship deriving unit 34.

The time-series data acquisition unit 32 acquires the volume of the compression band 10 based on the flow rate of the air supplied to the compression band 10 and the flow rate of the air discharged from the compression band 10 detected by the flow rate sensor 14. By using the flow rate of air, the volume can be acquired with a simple configuration.

The operation mode of the biological information measurement device 1 is set to a first mode or a second mode by the operation of the operator according to whether or not there is pulsation of the blood vessel of the subject. When there is pulsation, the first mode is set. When there is no pulsation, the second mode is set. Since pulsation also occurs in the internal pressure of the compression band 10 according to the pulsation of the blood vessel of the upper arm 80, the mode may be automatically set based on the amount of change in the internal pressure of the compression band 10.

In the first mode, the time-series data acquisition unit 32 acquires the internal pressure and the volume of the compression band 10 at each timing when the internal pressure of the compression band 10 becomes local maximum or local minimum. It can be said that the timing when the internal pressure becomes local maximum is the timing of the systolic blood pressure, and the timing when the internal pressure becomes local minimum is the timing of the diastolic blood pressure. Hereinafter, an example in which the internal pressure and the volume are acquired at the timing when the internal pressure becomes local minimum will be described, but the same applies to a case where the internal pressure and the volume are acquired at the timing when the internal pressure becomes local maximum.

In the second mode, while the internal pressure of the compression band 10 decreases, the time-series data acquisition unit 32 acquires the internal pressure and the volume of the compression band 10 at predetermined timings, for example, at regular time intervals.

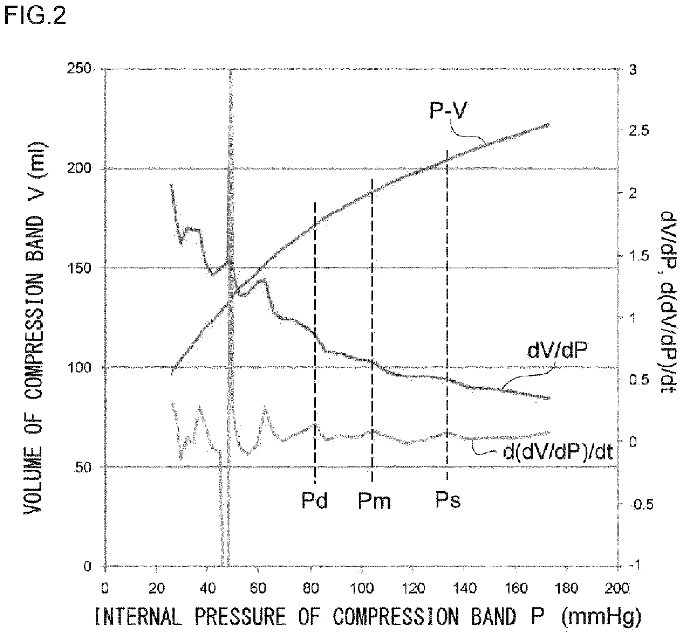

The relationship deriving unit 34 derives the relationship between the volume and the internal pressure of the compression band 10 as illustrated in Fig. 2 based on the time-series data of the volume and the internal pressure acquired by the time-series data acquisition unit 32 regardless of the mode. The relationship deriving unit 34 outputs the derived relationship between the volume and the internal pressure to the determination unit 36.

Fig. 2 illustrates a relationship between a volume and an internal pressure of the compression band 10 in Fig. 1. Fig. 2 illustrates an example of a relationship when there is pulsation in the internal pressure. While the internal pressure of the compression band 10 decreases from the target maximum pressure to the target minimum pressure, a plurality of sets of volume and internal pressure are acquired. The relationship deriving unit 34 derives a relationship between the volume and the internal pressure in Fig. 2, that is, a P-V characteristic that is a change in the volume with respect to a change in the internal pressure based on the plurality of sets of volume and internal pressure.

The relationship between the volume and the internal pressure includes information on the elasticity of the upper arm 80. As the internal pressure of the compression band 10 decreases from the target maximum pressure, the blood vessel of the upper arm 80 compressed by the compression band 10 opens, and the elasticity of the blood vessel changes. As a result, the elasticity of the upper arm 80 immediately below the compression band 10 also changes. Therefore, the relationship between the volume and the internal pressure includes information on a change in elasticity of the upper arm 80 accompanying the opening of the blood vessel.

When there is pulsation in the blood vessel, the blood vessel starts to open when the internal pressure of the compression band 10 reaches the systolic blood pressure. When the internal pressure of the compression band 10 reaches the diastolic blood pressure, the blood vessel opens almost completely. Therefore, in each of the systolic blood pressure and the diastolic blood pressure, a feature point at which the elasticity of the upper arm 80 changes appears in the relationship between the volume and the internal pressure. The feature point also appears in the average blood pressure. The feature point is a point at which a peak appears by differentiating the relationship between the volume and the internal pressure. Therefore, the systolic blood pressure, the diastolic blood pressure, and the average blood pressure can be determined by detecting the feature point.

When there is no pulsation in the blood vessel, the blood vessel opens when the internal pressure of the compression band 10 reaches the blood pressure of the subject. Therefore, a feature point appears in the relationship between the volume and the internal pressure in the blood pressure of the subject. Therefore, the blood pressure can be determined by detecting the feature point.

The determination unit 36 determines the blood pressure of the subject based on the peak position of the differential result of the relationship between the volume and the internal pressure derived by the relationship deriving unit 34. The determination unit 36 differentiates the relationship between the volume and the internal pressure illustrated in Fig. 2 with respect to the internal pressure to derive a first differential value $dV/dP$. Fig. 2 illustrates the relationship between the first differential value and the internal pressure. The determination unit 36 differentiates the first differential value with respect to time using the acquisition time of the internal pressure to derive a second differential value $d(dV/dP)/dt$. Fig. 2 illustrates the relationship between the second differential value and the internal pressure. The second differential value is obtained by removing a gradual change in the first differential value with respect to a change in the internal pressure, and includes a plurality of peaks. The determination unit 36 determines the internal pressure of the peak of the second differential value as the blood pressure.

In the first mode, when the second differential value includes a plurality of peaks as illustrated in Fig. 2, the determination unit 36 determines the highest internal pressure $Ps$ among the internal pressures of the plurality of peaks as the systolic blood pressure, determines the second highest internal pressure $Pm$ as the average blood pressure, and determines the third highest internal pressure Pd as the diastolic blood pressure. As a result, the systolic blood pressure or the like of a pulsating subject can be acquired.

In the second mode, when the relationship between the volume and the internal pressure includes a plurality of peaks, the determination unit 36 determines the highest internal pressure among the internal pressures of the plurality of peaks as the blood pressure. As a result, the blood pressure of the subject without pulsation can be acquired.

In order to remove the influence of noise, the determination unit 36 regards a local maximum point having a predetermined magnitude or more as a peak. The predetermined magnitude can be appropriately determined by experiments or the like. The determination unit 36 causes the display 26 to display the determined blood pressure.

According to the present embodiment, since the relationship between the volume and the internal pressure of the compression band 10 while the internal pressure of the compression band 10 decreases is derived, the relationship between the volume and the internal pressure includes a feature point at which the elasticity of the upper arm 80 changes due to the opening of the blood vessel. Then, since the internal pressure at the peak of the second differential value derived from the relationship between the volume and the internal pressure, that is, the internal pressure at the feature point is determined as the blood pressure, the blood pressure can be accurately and easily measured. Since the change in the amplitude of the pulse wave is not used, the blood pressure can be acquired with high accuracy even when the pulsation is weak and when there is no pulsation. Further, since the shape of the pulse wave is not acquired, the measurement accuracy of the blood pressure is less likely to decrease even if the internal pressure of the compression band is increased or decreased faster than the oscillometric method. Therefore, the blood pressure can be measured in a shorter time.

In the first mode, the internal pressure and the volume of the compression band 10 at each timing when the internal pressure of the compression band 10 becomes local maximum or local minimum are acquired so that the blood pressure of a pulsating subject can be acquired. In the second mode, since the internal pressure and the volume of the compression band 10 are acquired at every predetermined timing, the blood pressure of the subject without pulsation can be acquired.

Second Embodiment

In a second embodiment, FMD measurement is performed, and the dilation rate of the vascular diameter is acquired based on the internal pressure and the volume of the compression band 10. Hereinafter, differences from the first embodiment will be mainly described.

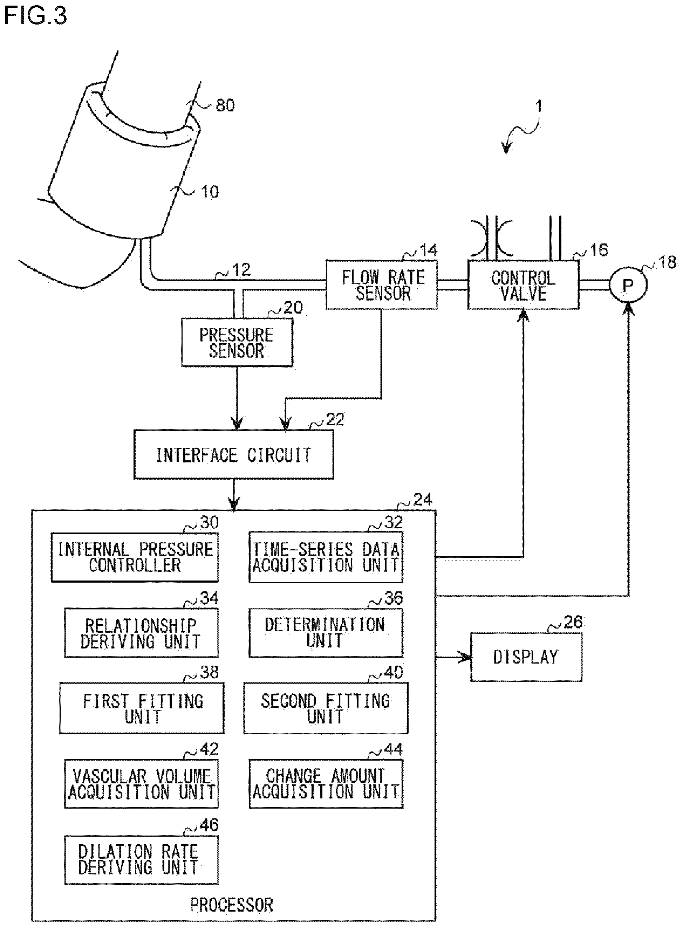

Fig. 3 illustrates a configuration of the biological information measurement device 1 according to the second embodiment. The biological information measurement device 1 measures a dilation rate of a vascular diameter as biological information by FMD measurement. The biological information measurement device 1 can also be referred to as a blood vessel function measurement device. The configuration of the processor 24 is different from that of the first embodiment. The processor 24 includes the internal pressure controller 30, the time-series data acquisition unit 32, the relationship deriving unit 34, the determination unit 36, a first fitting unit 38, a second fitting unit 40, a vascular volume acquisition unit 42, a change amount acquisition unit 44, and a dilation rate deriving unit 46.

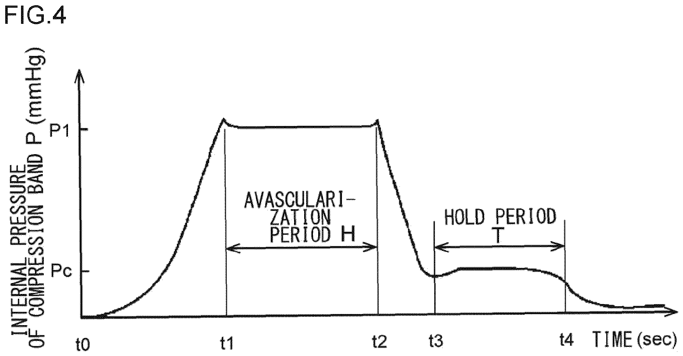

The internal pressure controller 30 maintains the internal pressure of the compression band 10 at an avascularization pressure P1 at which the upper arm 80 as a part of the living body is avascularized, and then reduces the internal pressure of the compression band 10 to a hold pressure Pc at which the avascularization is released. This will be described in detail with reference to Fig. 4.

Fig. 4 illustrates a temporal change in the internal pressure of the compression band 10 in Fig. 3. The internal pressure controller 30 starts increasing the internal pressure at time to, and when the internal pressure reaches the avascularization pressure P1 at time t1, the internal pressure controller 30 maintains the internal pressure at the avascularization pressure P1 for an avascularization period H. The avascularization pressure P1 is set to a value at which the artery is collapsed and closed, for example, about 180 mmHg. The avascularization period H is set to, for example, about several 10 seconds to several minutes.

The internal pressure controller 30 starts decreasing the internal pressure of the compression band 10 at time t2 after the avascularization period H has elapsed, and when the internal pressure reaches the hold pressure Pc at time t3, the internal pressure controller 30 causes the control valve 16 to stop the supply and discharge of air to maintain the internal pressure at the hold pressure Pc for a hold period T until time t4. The hold pressure Pc is set to a value at which the artery opens and the compression band 10 is in close contact with the upper arm, for example, about 15 to 20 mmHg.

When the blood flow in the artery resumes due to the release of the avascularization, nitric oxide (NO) is produced from the endothelium based on the shear stress of the blood flow acting on the endothelium of the artery, the smooth muscle is relaxed by the NO, and thereby a vasodilation response occurs. The hold period T is set to a period that sufficiently covers the timing at which the volume of the artery is maximized by the vasodilation response, for example, about 90 to 300 seconds.

The avascularization pressure P1, the avascularization period H, the hold pressure Pc, and the hold period T can be appropriately determined by experiments or the like.

The time-series data acquisition unit 32 acquires time-series data of the volume and the internal pressure of the compression band 10 while the internal pressure of the compression band 10 decreases from time t2 to t3. The time-series data acquisition unit 32 acquires the internal pressure and the volume of the compression band 10 at each timing when the internal pressure of the compression band 10 becomes local minimum.

The relationship deriving unit 34 derives the relationship between the volume and the internal pressure of the compression band 10 based on the time-series data acquired by the time-series data acquisition unit 32, and outputs the derived relationship between the volume and the internal pressure to the determination unit 36.

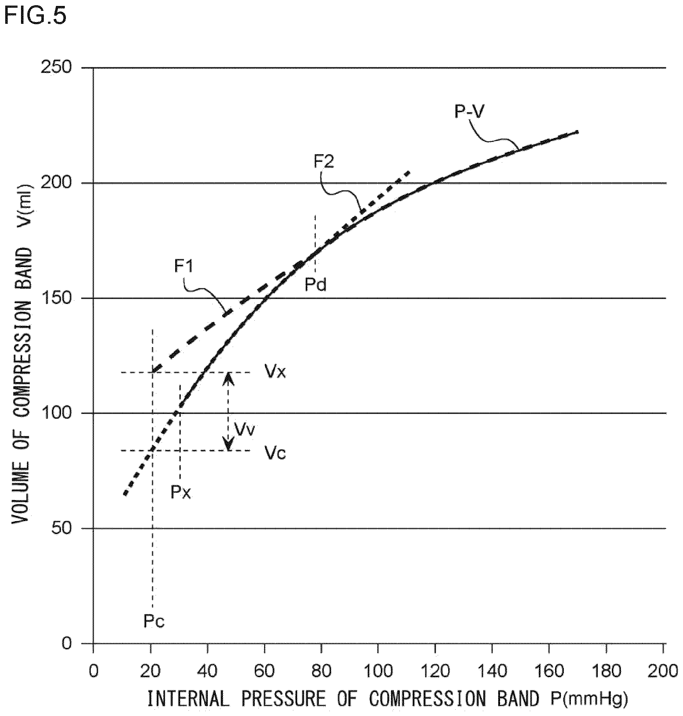

Fig. 5 illustrates a relationship between the volume and the internal pressure of the compression band 10 in Fig. 3, a first function F1, and a second function F2. Fig. 5 illustrates, as an example, the same relationship between the volume and the internal pressure as in Fig. 2.

As similar to the first embodiment, the determination unit 36 determines a diastolic blood pressure Pd of the living body based on the relationship between the volume and the internal pressure derived by the relationship deriving unit 34, and outputs the determined diastolic blood pressure Pd to the first fitting unit 38 and the second fitting unit 40.

The first fitting unit 38 fits the first function F1 to the relationship between the volume and the internal pressure derived by the relationship deriving unit 34 in a range where the internal pressure is higher than the diastolic blood pressure Pd determined by the determination unit 36. The first function F1 is, for example, a polynomial expressed by Formula (1). In the Formula (1), V represents a volume, and P represents an internal pressure. The first fitting unit 38 determines coefficients a, b, c of Formula (1) so that the correlation coefficient approaches 1 using a known technique. The first function F1 approximates the relationship between the volume and the internal pressure in a range where the internal pressure is higher than the diastolic blood pressure Pd.

$$V = a \times P^2 + b \times P + c \ \dots \ (1)$$

Since the internal pressure and the volume of the compression band 10 at each timing when the internal pressure becomes local minimum are acquired, the relationship between the volume and the internal pressure represents a state in which the artery and the vein are closed in a range where the internal pressure is higher than the diastolic blood pressure Pd. Therefore, the first function F1 also represents the relationship between the volume and the internal pressure in a state where the artery and the vein are closed.

The second fitting unit 40 fits the second function F2 to the relationship between the volume and the internal pressure derived by the relationship deriving unit 34 in a range where the internal pressure is equal to or higher than a predetermined pressure Px and equal to or lower than the diastolic blood pressure Pd. The pressure Px is higher than the hold pressure Pc. The second function F2 is, for example, a power function expressed by Formula (2). The second fitting unit 40 determines a coefficient d and an exponent e of Formula (2) so that the correlation coefficient approaches 1 using a known technique. The second function F2 approximates the relationship between the volume and the internal pressure in a range where the internal pressure is equal to or higher than the pressure Px and equal to or lower than the diastolic blood pressure Pd.

$$V = d \times P^e \ \dots \ (2)$$

The pressure Px is defined as a value at which the artery is opened and the vein is closed. Therefore, the relationship between the volume and the internal pressure represents a state in which the artery is opened and the vein is closed in a range where the internal pressure is equal to or higher than the pressure Px and equal to or lower than the diastolic blood pressure Pd. Therefore, the second function F2 also represents the relationship between the volume and the internal pressure in a state where the artery is opened and the vein is closed.

As the first function F1 and the second function F2, various convex upward functions can be used according to the relationship between the volume and the internal pressure. The first function F1 and the second function F2 may be known functions used for curve fitting, for example, a polynomial of the order of n (n is an integer of two or more) with respect to the internal pressure P, a formula including a trigonometric function (for example, sin P), a formula including a logarithmic function (for example, log P), or a spline function. The first function F1 and the second function F2 may be a function obtained by combining at least two of these functions including the Formulae (1) and (2). In addition, as in the above-described example, there may be a case where the formula of the first function F1 and the formula of the second function F2 are different from each other, or there may be a case where the formula of the first function F1 and the formula of the second function F2 are the same and coefficients and exponents are different from each other.

The vascular volume acquisition unit 42 acquires a value Vx of the first function F1 at the hold pressure Pc and a hold volume Vc which is a value of the second function F2 at the hold pressure Pc, and acquires a difference between the value Vx and the hold volume Vc as a vascular volume Vv. The vascular volume acquisition unit 42 outputs the acquired hold volume Vc and vascular volume Vv to the dilation rate deriving unit 46. The vascular volume Vv indicates the combined volume of the plurality of arteries of the upper arm 80 in a compression target region by the compression band 10.

The change amount acquisition unit 44 acquires time-series data of the internal pressure of the compression band 10 during the hold period T in Fig. 4, that is, while the internal pressure of the compression band 10 is maintained at the hold pressure Pc. The change amount acquisition unit 44 acquires the internal pressure at each timing when the internal pressure of the compression band 10 becomes local minimum.

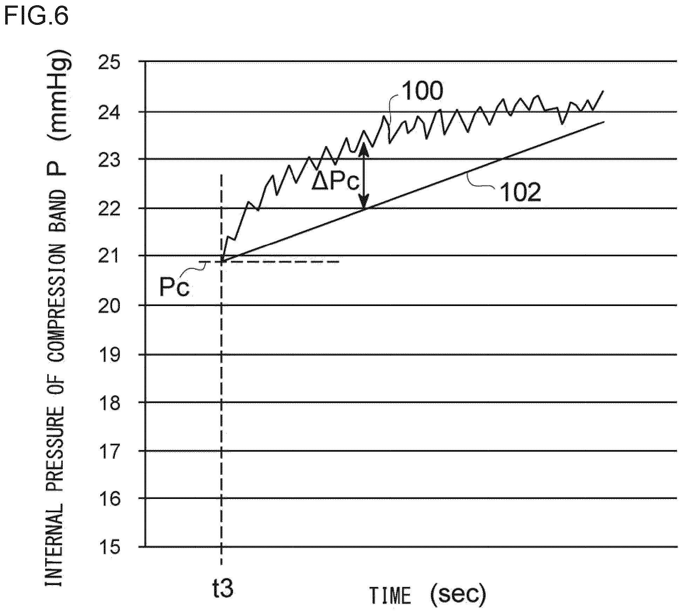

Fig. 6 illustrates a temporal change 100 from the hold pressure Pc of the internal pressure of the compression band 10 acquired by the change amount acquisition unit 44 and a characteristic 102 of the compression band 10. Fig. 6 illustrates a relationship between the internal pressure of the compression band 10 and time at and after time t3 in Fig. 4. The internal pressure of the compression band 10 vibrates due to the influence of the respiration of the subject.

When the artery expands due to the vasodilation response, the upper arm 80 bulges, this bulge increases the internal pressure of the compression band 10, and the internal pressure of the compression band 10 becomes higher than the hold pressure Pc as illustrated in Fig. 6. As a result, the volume of the compression band 10 becomes smaller than the hold volume Vc.

The change amount acquisition unit 44 acquires a change amount $\Delta Pc$ of the internal pressure of the compression band 10 after the internal pressure of the compression band 10 reaches the hold pressure Pc based on the acquired time-series data, and outputs the acquired change amount $\Delta Pc$ of the internal pressure to the dilation rate deriving unit 46.

Here, even in a case where the upper arm 80 does not exist, the compression band 10 in which the internal pressure is the hold pressure Pc has the characteristic 102 in which the internal pressure slightly increases with time. Therefore, the change amount acquisition unit 44 corrects the characteristic 102 of the compression band 10.

The change amount acquisition unit 44 derives the change amount $\Delta Pc$ of the internal pressure based on the difference between the temporal change 100 of the internal pressure of the compression band 10 after the internal pressure of the compression band 10 reaches the hold pressure Pc and the previously acquired characteristic 102 of the compression band 10 in which the internal pressure increases from the hold pressure Pc with time. The change amount acquisition unit 44 derives the maximum value of the difference as the change amount $\Delta Pc$ of the internal pressure. As a result, the change amount $\Delta Pc$ of the internal pressure caused by the vasodilation can be acquired more accurately.

The dilation rate deriving unit 46 derives the dilation rate R(%) of the vascular diameter of the living body based on the hold pressure Pc, the hold volume Vc, the vascular volume Vv, and the change amount $\Delta Pc$ of the internal pressure. The dilation rate deriving unit 46 derives $100 \times Vc \times \Delta Pc / (2Vv \times Pc)$ as the dilation rate R of the vascular diameter. The dilation rate deriving unit 46 causes the display 26 to display the derived dilation rate R of the vascular diameter.

Now, it will be described that the dilation rate R of the vascular diameter can be derived by the above formula.

When the internal pressure of the compression band 10 is the hold pressure Pc and the volume of the compression band 10 is the hold volume Vc, it is assumed that when the compression band 10 is deformed by the volume change $\Delta Vc$ of the upper arm 80 in the compression target region by the compression band 10 according to the vasodilation of the artery and the volume of the compression band 10 decreases by $\Delta Vc$, the internal pressure of the compression band 10 increases by $\Delta Pc$. However, it is assumed that all the volume change $\Delta Vc$ of the upper arm 80 is transmitted to the compression band 10 as the volume change of the compression band 10 and the temperature is constant. Since Formula (3) is established by Boyle's law, Formula (4) is established.

$$Pc \times Vc = \text{constant} \dots (3)$$

$$Pc \times Vc = (Pc + \Delta Pc) \times (Vc - \Delta Vc)$$
$$= Pc \times Vc + Vc \times \Delta Pc - Pc \times \Delta Vc - \Delta Pc \times \Delta Vc \dots (4)$$

$\Delta Pc \times \Delta Vc$ is a minute amount, and thus is omitted. Formula (4) is deformed as below:

$$Vc \times \Delta Pc - Pc \times \Delta Vc = 0 \dots (5),$$

and therefore,

$$Vc \times \Delta Pc = Pc \times \Delta Vc \ ... \ (6)$$

is established.

Accordingly, Formula (7) is obtained.

$$\Delta Vc = (Vc/Pc) \times \Delta Pc \ ... \ (7)$$

From Formula (7), $\Delta Vc$ is obtained by measuring $Vc$, $Pc$, and $\Delta Pc$.

Here, a change rate $\Delta Vc/Vv$, which is a ratio of the above-described volume change $\Delta Vc$ to the vascular volume $Vv$ of the upper arm 80 in the compression target region by the compression band 10, is expressed by Formula (8). It is assumed that the volume change $\Delta Vc$ is a volume change of the blood vessel, where $D$ is the radius of the combined blood vessel of the plurality of arteries in the vascular bed in the compression target region by the compression band 10, and $L$ is the length of the compression target region. The radius $D$ of the combined blood vessel is the radius of the cylinder with the combined volume of the plurality of arteries in which a volume change is considered to occur in the compression target region.

$$\begin{aligned} \Delta Vc \ / \ Vv \ &= \pi \ ( \ (D + \Delta D)^2 - D^2) \ L \ / \ \pi D^2 L \\ &= (2D\Delta D + \Delta D^2) \ / \ D^2 \\ &= (2D + \Delta D) \ \Delta D \ / \ D^2 \\ &\approx 2 \ D\Delta D/D^2 \\ &= 2 \ \Delta D/D \ ... \ (8) \end{aligned}$$

The dilation rate $R$ (%) of the vascular diameter is defined by Formula (9).

$$R = 100 \times \Delta D/D \ ... \ (9)$$

When Formula (8) is substituted into Formula (9), Formula (10) is obtained.

$$R = 100 \times \Delta Vc \ / \ 2Vv \ ... \ (10)$$

When Formula (7) is substituted into Formula (10), Formula (11) that is the above-described formula is obtained.

$$R = 100 \times Vc \times \Delta Pc \ / \ (2 \times Vv \times Pc) \ ... \ (11)$$

According to the present embodiment, the first function F1 and the second function F2 are fitted to the relationship between the volume and the internal pressure of the compression band 10 separately by the diastolic blood pressure Pd, and the difference between the value $Vx$ of the first function F1 at the hold pressure $Pc$ and the value (hold volume $Vc$) of the second function F2 at the hold pressure $Pc$ is acquired as the vascular volume $Vv$. Thus, the vascular volume $Vv$ of the upper arm 80 can be accurately acquired. Since the hold pressure $Pc$ and the change amount $\Delta Pc$ of the internal pressure can be acquired, the dilation rate $R$ of the vascular diameter can be accurately measured based on these values and Formula (11).

Since the diastolic blood pressure Pd is determined in the same manner as in the first embodiment, the accuracy is high. Therefore, the accuracy of the value $Vx$ and the hold volume $Vc$ also increases, and the accuracy of the vascular volume $Vv$ also increases.

Further, since the dilation rate $R$ of the vascular diameter is measured by wrapping the compression band 10 around a part of the living body, measurement is easy and can be performed in a short time without requiring a special measurement technique as compared with an existing apparatus using an ultrasound image. Since the dilation rate $R$ of the vascular diameter reflecting the information on the volume change of the plurality of arteries in the compression target region of the compression band 10 can be acquired, it is easy to acquire the dilation rate $R$ of the vascular diameter with high reproducibility. That is, it is possible to avoid a variation in the dilation rate of the vascular diameter according to the vascular diameter or the measurement position of the artery to be measured, which is likely to occur in an existing FMD measurement using an ultrasound image. Further, since the change rate ΔVc/Vv of the vascular volume Vv is converted into the dilation rate R of the vascular diameter, the dilation rate of the vascular diameter obtained by the existing FMD measurement using the ultrasound image can be compared with the measured value of the present embodiment.

Furthermore, as compared with the existing device using the ultrasound image, an ultrasound probe, a probe support device that supports the ultrasound probe and searches for an optimum position, an ultrasound image generation device that generates an ultrasound image, and the like are unnecessary. Therefore, the configuration can be simplified, and the biological information measurement device 1 can be reduced in cost and size.

Moreover, there is a possibility that some veins are also opened at the time of the hold pressure Pc. However, since the hold volume Vc at the time of the hold pressure Pc is acquired based on the second function F2 fitted in a range equal to or higher than the pressure Px higher than the hold pressure Pc, it is easy to acquire the hold volume Vc not including the volume of the veins. Since the vasodilation response does not occur in the vein, the hold volume Vc is close to the volume of the artery, so that a more accurate dilation rate R of the vascular diameter can be acquired. Since the hold pressure Pc does not have to be a strict value at which the vein is closed, the hold pressure Pc can be easily set.

Third Embodiment

A third embodiment is different from the second embodiment in that the second function F2 is not used and the measured value of the volume of the compression band 10 at the hold pressure Pc is set to the hold volume Vc. Hereinafter, differences from the second embodiment will be mainly described.

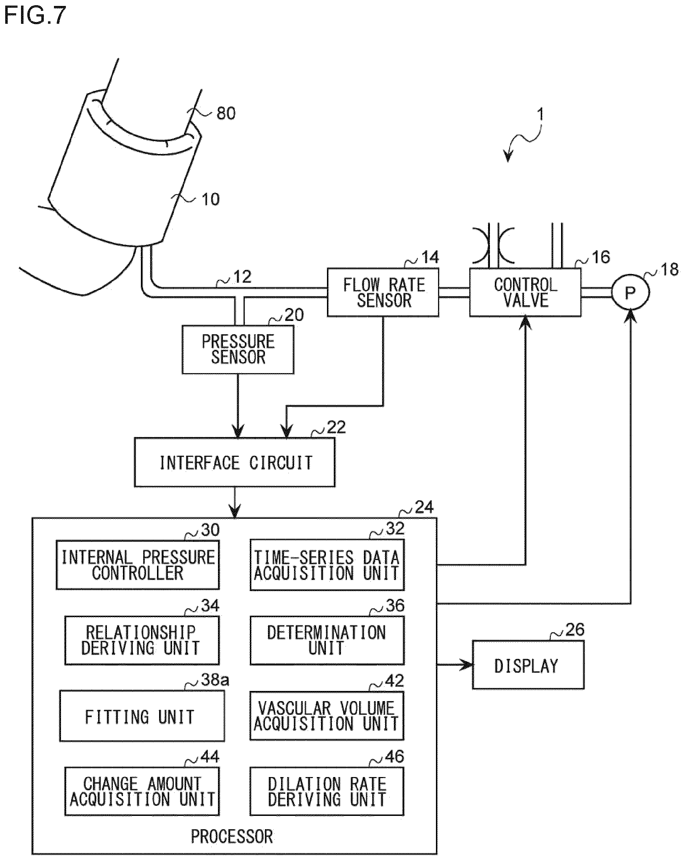

Fig. 7 illustrates a configuration of the biological information measurement device 1 according to the third embodiment. The configuration of the processor 24 is different from that of the second embodiment, and the second fitting unit is not provided. A fitting unit 38a has the same function as that of the first fitting unit 38 of the second embodiment. The first function F1 of the second embodiment is referred to as a function F.

The vascular volume acquisition unit 42 acquires the hold volume Vc that is the volume at the hold pressure Pc in the relationship between the volume and the internal pressure derived by the relationship deriving unit 34, and acquires the difference between the value Vx of the function F at the hold pressure Pc and the hold volume Vc as the vascular volume Vv.

In a case where some veins are opened at the hold pressure Pc, since the hold volume Vc also includes the volume of the opened veins, there is a possibility that the accuracy of the dilation rate R of the vascular diameter is lower than that of the second embodiment. Therefore, in order to increase the accuracy, it is preferable to set the hold pressure Pc to a value at which the artery are opened and the vein are closed.

According to the present embodiment, since only one type of function needs to be fitted, the processing of the processor 24 can be simplified as compared with the second embodiment.

The present disclosure has been described above based on the embodiments. These embodiments are examples, and it is understood by those skilled in the art that various modifications can be made to the combinations of the respective constituent elements and the respective processing processes, and such modifications are also within the scope of the present disclosure.

For example, in the first embodiment, the time-series data acquisition unit 32 may acquire the internal pressure and the volume of the compression band 10 at each timing when the internal pressure of the compression band 10 becomes local maximum and local minimum. The relationship deriving unit 34 may derive the first relationship between the volume and the internal pressure of the compression band 10 based on the time-series data at the timing when the internal pressure of the compression band 10 becomes local minimum, and derive the second relationship between the volume and the internal pressure of the compression band 10 based on the time-series data at the timing when the internal pressure of the compression band 10 becomes local maximum. The determination unit 36 may determine the diastolic blood pressure based on the peak position of the differential result of the first relationship and determine the systolic blood pressure based on the peak position of the differential result of the second relationship. In this modification, it is easy to accurately measure the blood pressure even when the influence of noise is large.

In the first to third embodiments, the time-series data acquisition unit 32 may acquire the time-series data of the volume and the internal pressure of the compression band 10 while the internal pressure increases from around 0 instead of while the internal pressure of the compression band 10 decreases. In this modification, the degree of freedom of the configuration of the biological information measurement device 1 can be improved.

In the first embodiment, the first fitting unit 38 and the second fitting unit 40 of the second embodiment may be provided. The determination unit 36 may set the internal pressure at the intersection of the first function F1 and the second function F2 as the diastolic blood pressure. In this modification, even when the value between the two internal pressures acquired by the time-series data acquisition unit 32 is the systolic blood pressure, a more accurate diastolic blood pressure can be acquired.

In the first embodiment, the determination unit 36 may derive the second differential value by differentiating the first differential value with respect to the internal pressure. In this modification, the degree of freedom of the configuration of the biological information measurement device 1 can be improved.

In the first to third embodiments, the compression band 10 is wrapped around the upper arm 80, but may be wrapped around a lower limb, a finger, a wrist, or the like.

In the second and third embodiments, the dilation rate deriving unit 46 may derive the dilation rate R'(%) of the vascular volume Vv instead of the dilation rate R of the vascular diameter. Since the dilation rate R' of the vascular volume Vv is defined by R' = 100 × ΔVc/Vv, the dilation rate R' is expressed by Formula (12) by substituting Formula (7) into this formula.

$$R' = 100 \times Vc \times \Delta Pc\ /\ (Vv \times Pc) \ \dots \ (12)$$

The dilation rate deriving unit 46 derives 100 × Vc × ΔPc / (Vv×Pc) as the dilation rate R' of the vascular volume Vv. In this modification, another index for the vasodilation response can be obtained.

REFERENCE SIGNS LIST

1 biological information measurement device, 10 compression band, 14 flow rate sensor, 30 internal pressure controller, 32 time-series data acquisition unit, 34 relationship deriving unit, 36 determination unit, 38 first fitting unit, 38a fitting unit, 40 second fitting unit, 42 vascular volume acquisition unit, 44 change amount acquisition unit, 46 dilation rate deriving unit

INDUSTRIAL APPLICABILITY

The present disclosure can be used for a technique for measuring biological information.

**Claims**

**1.** A biological information measurement device comprising:

an internal pressure controller that increases or decreases an internal pressure of a compression band wrapped around a part of a living body;
a time-series data acquisition unit that acquires time-series data of a volume and an internal pressure of the compression band while the internal pressure of the compression band increases or decreases;
a relationship deriving unit that derives a relationship between the volume and the internal pressure of the compression band based on the time-series data; and
a determination unit that differentiates the relationship with respect to the internal pressure to derive a first differential value, differentiates the first differential value with respect to time or the internal pressure to derive a second differential value, and determines an internal pressure at a peak of the second differential value as a blood pressure of the living body.

**2.** The biological information measurement device according to claim 1, wherein, when the second differential value includes a plurality of peaks, the determination unit determines the highest internal pressure among internal pressures of the plurality of peaks as a systolic blood pressure, determines a second highest internal pressure as an average blood pressure, and determines a third highest internal pressure as a diastolic blood pressure.

**3.** The biological information measurement device according to claim 1 or 2, wherein the time-series data acquisition unit acquires an internal pressure and a volume of the compression band at each timing when the internal pressure of the compression band becomes local maximum or local minimum.

**4.** The biological information measurement device according to claim 1, wherein the time-series data acquisition unit acquires the internal pressure and the volume of the compression band at each predetermined timing.

**5.** The biological information measurement device according to any one of claims 1 to 4, wherein the internal pressure controller supplies gas to the compression band to increase the internal pressure, and discharges the gas from the compression band to decrease the internal pressure, and
the time-series data acquisition unit acquires the volume of the compression band based on a flow rate of the gas supplied to the compression band and the flow rate of the gas discharged from the compression band detected by a flow rate sensor.

6. A biological information measurement device comprising:

an internal pressure controller that increases an internal pressure of a compression band wrapped around a part of a living body, maintains the internal pressure at an avascularization pressure at which the part of the living body is subjected to avascularization, and then lowers the internal pressure to a hold pressure at which avascularization is released;
a time-series data acquisition unit that acquires time-series data of a volume and the internal pressure of the compression band while the internal pressure of the compression band increases or decreases;
a relationship deriving unit that derives a relationship between the volume and the internal pressure of the compression band based on the time-series data;
a first fitting unit that fits a first function to the relationship in a range in which the internal pressure is higher than a diastolic blood pressure of the living body;
a second fitting unit that fits a second function to the relationship in a range in which the internal pressure is equal to or higher than a predetermined pressure higher than the hold pressure and equal to or lower than the diastolic blood pressure;
a vascular volume acquisition unit that acquires, as a vascular volume, a difference between a value of the first function at the hold pressure and a hold volume that is a value of the second function at the hold pressure;
a change amount acquisition unit that acquires an amount of change in the internal pressure of the compression band after the internal pressure of the compression band reaches the hold pressure; and
a dilation rate deriving unit that derives a dilation rate of a vascular diameter or the vascular volume of the living body based on the hold pressure, the hold volume, the vascular volume, and the amount of change in the internal pressure.

7. A biological information measurement device comprising:

an internal pressure controller that increases an internal pressure of a compression band wrapped around a part of a living body, maintains the internal pressure at an avascularization pressure at which the part of the living body is subjected to avascularization, and then lowers the internal pressure to a hold pressure at which avascularization is released;
a time-series data acquisition unit that acquires time-series data of a volume and the internal pressure of the compression band while the internal pressure of the compression band increases or decreases;
a relationship deriving unit that derives a relationship between the volume and the internal pressure of the compression band based on the time-series data;
a fitting unit that fits a function to the relationship in a range in which the internal pressure is higher than a diastolic blood pressure of the living body;
a vascular volume acquisition unit that acquires, as a vascular volume, a difference between a value of the function at the hold pressure and a hold volume that is a volume at the hold pressure in the relationship;
a change amount acquisition unit that acquires an amount of change in the internal pressure of the compression band after the internal pressure of the compression band reaches the hold pressure; and
a dilation rate deriving unit that derives a dilation rate of a vascular diameter or the vascular volume of the living body based on the hold pressure, the hold volume, the vascular volume, and the amount of change in the internal pressure.

8. The biological information measurement device according to claim 6 or 7, wherein, when Vc represents the hold volume, Pc represents the hold pressure, Vv represents the vascular volume, and $\Delta Pc$ represents the amount of change in the internal pressure,
the dilation rate deriving unit derives $100 \times Vc \times \Delta Pc / (2Vv \times Pc)$ as the dilation rate of the vascular diameter.

9. The biological information measurement device according to any one of claims 6 to 8, wherein the change amount acquisition unit derives the amount of change in the internal pressure based on a difference between a temporal change in the internal pressure of the compression band after the internal pressure of the compression band reaches the hold pressure and a characteristic of the compression band acquired in advance in which the internal pressure increases from the hold pressure with time.

10. The biological information measurement device according to any one of claims 6 to 9, further comprising a determination unit that differentiates the relationship with respect to an internal pressure to derive a first differential value, differentiates the first differential value with respect to time or the internal pressure to derive a second differential value, and determines the diastolic blood pressure based on an internal pressure at a peak of the second differential value.

11. The biological information measurement device according to any one of claims 6 to 10, wherein the time-series data acquisition unit acquires the internal pressure and the volume of the compression band at each timing when the internal pressure of the compression band becomes local minimum.

FIG.1

FIG.2

## FIG.3

FIG.4

FIG.5

FIG.6

## FIG.7

PROCESSOR block containing:
- 30 INTERNAL PRESSURE CONTROLLER
- 32 TIME-SERIES DATA ACQUISITION UNIT
- 34 RELATIONSHIP DERIVING UNIT
- 36 DETERMINATION UNIT
- 38a FITTING UNIT
- 42 VASCULAR VOLUME ACQUISITION UNIT
- 44 CHANGE AMOUNT ACQUISITION UNIT
- 46 DILATION RATE DERIVING UNIT

Other elements: 80, 10, 12, 14 FLOW RATE SENSOR, 16 CONTROL VALVE, 18 P, 20 PRESSURE SENSOR, 22 INTERFACE CIRCUIT, 24, 26 DISPLAY, 1

## INTERNATIONAL SEARCH REPORT

| International application No. |
|---|
| PCT/JP2018/045306 |

**A. CLASSIFICATION OF SUBJECT MATTER**
Int. Cl. A61B5/022(2006.01)i

According to International Patent Classification (IPC) or to both national classification and IPC

**B. FIELDS SEARCHED**

Minimum documentation searched (classification system followed by classification symbols)
Int. Cl. A61B5/022

Documentation searched other than minimum documentation to the extent that such documents are included in the fields searched
Published examined utility model applications of Japan 1922-1996
Published unexamined utility model applications of Japan 1971-2019
Registered utility model specifications of Japan 1996-2019
Published registered utility model applications of Japan 1994-2019

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2017-148195 A (YUNEKUSU KK) 31 August 2017, entire text, all drawings (Family: none) | 1-11 |
| A | JP 2016-220886 A (NATIONAL UNIVERSITY CORPORATION KANAZAWA UNIVERSITY) 28 December 2016, entire text, all drawings (Family: none) | 1-11 |
| A | JP 2009-219623 A (OMRON HEALTHCARE CO., LTD.) 01 October 2009, entire text, all drawings & US 2011/0021927 A1 & CN 101969839 A | 1-11 |
| A | JP 2008-228934 A (NATIONAL INSTITUTE OF ADVANCED INDUSTRIAL SCIENCE AND TECHNOLOGY) 02 October 2008, entire text, all drawings (Family: none) | 1-11 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13.02.2019 | 26.02.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**EP 3 895 608 A1**

<table>
<tr><td colspan="2" align="center"><strong>INTERNATIONAL SEARCH REPORT</strong></td><td>International application No.<br>PCT/JP2018/045306</td></tr>
</table>

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2011-239972 A (SEIKO EPSON CORP.) 01 December 2011, entire text, all drawings & CN 102247169 A | 1-11 |

Form PCT/ISA/210 (continuation of second sheet) (January 2015)

22

**INTERNATIONAL SEARCH REPORT**

| International application No. |
|---|
| PCT/JP2018/045306 |

**Box No. II        Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:

    [see extra sheet]

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.

PCT/JP2018/045306

(continuation of Box No. III)

Document 1: JP 2017-148195 A (YUNEKUSU KK) 31 August 2017, entire text, all drawings (Family: none)

　　The claims are classified into the following two inventions.

　　(Invention 1) Claims 1-5
　　The above claims have the special technical feature of "a biological information measurement device, characterized by comprising: an internal pressure control unit for increasing or decreasing the internal pressure of a compression band that has been wound about a part of a body; a time series data acquisition unit for acquiring time series data for the volume and internal pressure of said compression band while the internal pressure of the compression band is increasing/decreasing; a relationship derivation unit for deriving the relationship between the volume and the internal pressure of the compression band on the basis of the time series data; and a determination unit that differentiates the relationship by the internal pressure to derive a first differential value, differentiates said first differential value by the time or internal pressure to derive a second differential value, and determines the internal pressure of a peak of said second differential value to be the blood pressure of the body," and are therefore classified as invention 1.

　　(Invention 2) Claims 6-11
　　The above claims share with claim 1 classified as invention 1 the feature of a "biological information measurement device, characterized by comprising: an internal pressure control unit for increasing or decreasing the internal pressure of a compression band that has been wound about a part of a body; a time series data acquisition unit for acquiring time series data for the volume and internal pressure of said compression band while the internal pressure of the compression band is increasing/decreasing; and a relationship derivation unit for deriving the relationship between the volume and the internal pressure of the compression band on the basis of the time series data."
　　However, this feature does not make a contribution over the prior art in the light of the content disclosed in document 1 (in particular, document 1 (paragraphs [0023]-[0031] and fig. 2, 3, and 5 discloses an endothelial function examination device which has a compression pressure control unit for increasing/reducing the internal pressure of a compression band that has been wound about a part of a body, and which experimentally finds the relationship between a compression pressure PC of the compression band and a volume V of the compression band), and therefore cannot be said to be a special technical feature. Moreover, no other identical or corresponding special technical feature exists between the above claims and claim 1.

Form PCT/ISA/210 (extra sheet) (January 2015)

**INTERNATIONAL SEARCH REPORT**

| International application No. |
| --- |
| PCT/JP2018/045306 |

    In addition, the above claims are not dependent claims of claim 1. Furthermore, the above claims are not substantially identical to or similarly closely related to any of the claims classified as invention 1.

    As such, the above claims cannot be classified as invention 1.

Form PCT/ISA/210 (extra sheet) (January 2015)

**REFERENCES CITED IN THE DESCRIPTION**

**Patent documents cited in the description**

- JP 2015009044 A